# EUROPEAN PATENT SPECIFICATION

(11) **EP 1 934 252 B1**
(45) Date of publication and mention of the grant of the patent: **24.06.2015**
(21) Application number: 05813799.3
(22) Date of filing: 13.10.2005
(51) Int. Cl.: C07K 14/62

(54) **PROCESS FOR THE PREPARATION OF INSULIN CONJUGATES.**
VERFAHREN ZUR HERSTELLUNG VON INSULINKONJUGATEN
PROCEDEDE SYNTHESE DE CONJUGUES D'INSULINE

(43) Date of publication of application: 25.06.2008
(73) Proprietor: Biocon Limited, Bangalore 560 100 Karnataka (IN)
(72) Inventor: DAVE, Nitesh, Karnataka 560100 (IN); HAZRA, Partha, Karnataka 560100 (IN); GOEL, Anuj, Karnataka 560100 (IN); ROY, Nita, Karnataka 560100 (IN); KHEDKAR, Anand, Karnataka 560100 (IN); IYER, Harish, Karnataka 560100 (IN); KRISHNAN, Gautam, Karnataka 560100 (IN); MANJUNATH, H. S., Karnataka 560100 (IN); SURYANARAYAN, Shrikumar, Karnataka 560100 (IN); MANIKAM, Govindasamy, Karnataka 560100 (IN); SACHDEV, Goldy, Karnataka 560100 (IN); GARG, Mayank, Karnataka 560100 (IN)
(74) Representative: Chalk, Anthony John
(86) International application number: PCT/IN2005/000338
(87) International publication number: WO 2007/043059

(56) References cited:
- WO-A1-02/098232
- WO-A1-2004/044206
- WO-A1-2005/016312
- WO-A1-2007/007345
- WO-A2-03/022208
- WO-A2-03/022996
- WO-A2-2004/083234
- WO-A2-2004/083234
- DING JIN-GUO ET AL: "Expression of monomeric insulin precursor in Pichia pastoris and its conversion into monomeric B27 Lys destripeptide insulin by tryptic hydrolysis" ACTA BIOCHIMICA ET BIOPHYSICA SINICA, BLACKWELL PUBLISHING, INC., MALDEN, MA, US LNKD- DOI:10.1111/J.1745-7270.2005.00040.X, vol. 37, no. 4, 1 April 2005 (2005-04-01), pages 234-240, XP009134280 ISSN: 1672-9145
- KJELDSEN T ET AL: "SECRETORY EXPRESSION AND CHARACTERIZATION OF INSULIN IN PICHIA PASTORIS" BIOTECHNOLOGY AND APPLIED BIOCHEMISTRY, ACADEMIC PRESS, US, vol. 29, 1 January 1999 (1999-01-01), pages 79-86, XP001070797 ISSN: 0885-4513
- STILL J.G.: 'Development of Oral Insulin: Progress and Current Status' DIABETES/METABOLISM RESEARCH AND REVIEWS vol. 18, 2002, pages S29 - S37, XP003011705
- WANG YAN ET AL: "Human insulin from a precursor overexpressed in the methylotrophic yeast Pichia pastoris and a simple procedure for purifying the expression product", BIOTECHNOLOGY AND BIOENGINEERING, WILEY & SONS, HOBOKEN, NJ, US, vol. 73, no. 1, 5 April 2001 (2001-04-05), pages 74-79, XP002429621, ISSN: 0006-3592, DOI: 10.1002/1097-0290(20010405)73:1<74::AID-BI T1038>3.0.CO;2-V

## Description

### FIELD OF THE INVENTION

The present invention relates to a process for an insulin-oligomer conjugate of formula insulin-oligomer. The process involves cloning and expression of an precursor IP-F of formula G-A-V-R-[B-Chain]-R-D-A-D-D-R-[A-Chain] and subjecting the expressed biosynthetic precursor to conjugation with an activated oligomer followed by protease treatment and purification to get an active insulin-oligomer conjugate.

### BACKGROUND OF THE INVENTION

The β-cells of the pancreatic islets secrete a single chain precursor of insulin, known as pro-insulin which upon proteolysis results in the biologically active polypeptide insulin. The insulin molecule is a highly conserved across species and generally consists of two chains of amino adds linked by disulfide bonds. The natural human insulin molecule (mw 5,807 Daltons), has A-chain of 21 amino add residues with glycine at the amino terminus; and a B-chain of 30 amino acid residues with phenylalanine at the amino terminus. Insulin may exist as a monomer or may aggregate into a dimer or a hexamer formed from three of the dimers. The monomer has the ability to bind to receptors and is the biologically active form.

Insulin polypeptide is the primary hormone responsible for controlling the transport, utilization and storage of glucose in the body. A defect in the carbohydrate metabolism as a result of insufficient production of insulin or reduced sensitivity of the receptor to insulin leads to the biological disorder diabetes. Diabetes impairs the normal ability to use glucose as a result increases blood sugar levels (hyperglycemia). As glucose accumulates in the blood, excess levels of sugar are excreted in the urine (glycosuria). Other symptoms of diabetes include increased urinary volume and frequency, thirst, itching, hunger, weight loss, and weakness. Diabetes when left untreated leads to ketosis, followed by acidosis with nausea and vomiting. As the toxic products continue to build up, the patient goes into a diabetic coma, which leads to the patient's death. There are two types of diabetes. Type I is insulin-dependent diabetes mellitus, or IDDM. IDDM was formerly referred to as "juvenile onset diabetes." In IDDM, insulin is not secreted by the pancreas and must be provided from an external source. Type II or adult-onset diabetes can ordinarily be controlled by diet, although in some advanced cases insulin is required.

Traditionally bovine and porcine insulin were used almost exclusively to treat diabetes in humans. With the development of recombinant technology commercial scale manufacture of human insulin was made possible by fermentation. Furthermore, genetically engineered insulin analogs having biological activity comparable to that of natural human insulin were developed to combat the disease. However, treatment of diabetes typically requires regular injections of insulin. Due to the inconvenience of insulin injections, various approaches have been attempted to formulate insulin for administration by non-injectable routes. A list of such publications include: US 4,338,306 (Kitao et al.) reports a pharmaceutical compositions of insulin and fatty acids having 8 to 14 carbon atoms and nontoxic salts thereof for rectal administration of insulin; US 4,579,730 (Kidron et al) reports an enterocoated insulin compositions with a bile acid or alkali metal salt thereof for the oral administration of insulin; US 5,283,236 (Chiou et al.) reports an insulin composition with a permeation-enhancing agent to aid systemic absorption of higher molecular weight polypeptides, as well as peptidase inhibitors for systemic delivery of insulin through the eyes wherein the drug passes into the nasolacrimal duct and becomes absorbed into circulation; US 5,658,878 (Backstrom et al.) reports an insulin and sodium salt of a saturated fatty acid of carbon chain length 10 (i.e., sodium caprate), 12 (sodium laurate), or 14 (sodium myristate) which enhances the absorption of insulin in the lower respiratory tract; US 5,853,748 (New et al.)- reports an enteric-coated composition of insulin, a bile salt or bile acid, and carbonate or bicarbonate ions, used to adjust the pH of the gut to a pH of from 7.5 to 9 for the oral administration of insulin. US 6,200,602 (Watts et al) reports a drug delivery composition of insulin for colonic delivery of insulin with an absorption promoter which includes a mixture of fatty acids having 6 to 16 carbon atoms and its salts or a mixture of mono/diglycerides of medium chain fatty acids along with a dispersing agent, in a coating to prevent the release of the insulin and absorption promoter until the tablet, capsule or pellet reaches the proximal colon.

Several attempts to deliver insulin by oral administration are found in literature. The problems associated with oral administration of insulin to achieve euglycemia in diabetic patients are well documented in pharmaceutical and medical literature. Digestive enzymes in the GI tract rapidly degrade insulin, resulting in biologically breakdown products. In the stomach, for example, orally administered insulin undergoes enzymatic proteolysis and acidic degradation. Survival in the intestine is hindered by excessive proteolysis. In the lumen, insulin is barraged by a variety of enzymes including gastric and pancreatic enzymes, exo- and endopeptidases, and brush border peptidases. Even if insulin survives this enzymatic attack, the biological barriers that must be traversed before insulin can reach its receptors in vivo may limit oral administration of insulin. For example, insulin may possess low membrane permeability, limiting its ability to pass from the lumen into the bloodstream.

Pharmaceutically active polypeptides such as insulin have been conjugated with polydispersed mixtures of polyethylene glycol or polydispersed mixtures of polyethylene glycol containing polymers to provide polydispersed mixtures of drug-oligomer conjugates; US 4,179,337 (Davis et al) reports conjugating polypeptides such as insulin with various polyethylene glycols such as MPEG-1900 and MPEG-5000 supplied by Union Carbide. US 5,567,422 (Greenwald) reports the conjugation of biologically active nucleophiles with polyethylene glycols such as m-PEG-OH (Union Carbide), which has a number average molecular weight of 5,000 Daltons.

Conjugation of polypeptides such as insulin with polyethylene glycol modified glycolipid polymers and polyethylene glycol modified fatty acid polymers are reported in US 5,359,030 (Ekwuribe et al.)*.*

US 6,011,008 (Domb et al.) reports a method for producing a water-soluble polysaccharide conjugate of an oxidation-sensitive substance comprising activating the polysaccharide to a dialdehyde by periodate oxidation; (b) purifying the dialdehyde from interfering anions and by-products; and (c) coupling the substance to the purified dialdehyde by Schiff base formation to form the conjugate. Optionally, the conjugate of step (c) is reduced to an amine conjugate by a reducing substance. Insulin was conjugated to oxidized AG (arabinogalactan) via an amine or imine bond by reacting a solution of pure oxidized AG (arabinogalactan) in borate buffer solution at pH 8.9 with insulin at 4°C overnight. The clear solution was dialyzed through a cellulose dialysis and the solution was lyophilized to yield 115 mg of a white solid.

US 6,022,524 (Maisano et al.) reported conjugation of Gd-DTPA with porcine insulin in a solution of DTPA and dimethylsulfoxide (DMSO) which was prepared by heating and stirring, then cooled at room temperature and added with a solution of 11.73 g NHS (0.102 mol) in 300 ml DMSO, then, drop by drop, with a solution of 19.6 g of N,N'-dicyclohexylcarbodiimide (0.097 mol) in 400 ml DMSO. The mixture is stirred for 16 hours, then filtered and the filtrate is concentrated by evaporation at 50. Degree.C and 5 Pa to a thick oil of an about 160 ml volume.

U.S. Pat. No. 6,309,633 (Ekwuribe et al.)--reports the use of solid insulin for conjugation of insulin with laurate PEG₅ in presence of Triethylamine and DMSO at room temperature. The reaction was monitored via HPLC every 30 mins. The conjugate was purified using a preparative HPLC.

U.S. Pat. No. 6,828,297 (Ekwuribe et al.) reports methods for making PEG7-Hexyl-Insulin by using zinc or zinc free human insulin for conjugation with activated oligomer and purification of B29 modified PEG7-Hexyl-Insulin, insulin in dimethylsulfoxide and triethyl amine was reacted with activated oligomer at 22+/-4°C. The crude reaction mixture is dialyzed or difiltered to remove organic solvents and small molecular weight impurities, exchanged against ammonium acetate buffer and lyophilized; which is further subjected to RP-HPLC equilibrated with 0.5% triethylamine/0.5% phosphoric acid buffer (TEAP A). The column was eluted with a gradient flow using TEAP A and TEAP B (80% acetonitrile and 20% TEAP A) solvent system. Fractions containing the conjugate were pooled and the elution buffer and solvent were removed by dialysis or diafiltration against ammonium acetate buffer and lyophilized to produce white powder of PEG7-hexyl-insulin, B29 monoconjugate (purity>97%). Currently, the existing prior art teaches use of pure insulin powder or crystals as the starting material for making conjugated insulin wherein the insulin used is a biologically active form.

WO-A-2004/083234 discloses methods for synthesizing proinsulin polypeptides that include contacting a proinsulin polypeptide including an insulin polypeptide coupled to one or more peptides by peptide bond(s) capable of being cleaved to yield the insulin polypeptide with an oligomer under conditions sufficient to couple the oligomer to the insulin polypeptide portion of the proinsulin polypeptide and provide a proinsulin polypeptide-oligomer conjugate, and cleaving the one or more peptides from the proinsulin polypeptide-oligomer conjugate to provide the insulin polypeptide-oligomer conjugate. Methods of synthesizing proinsulin polypeptide-oligomer conjugates, C-peptide polypeptide-oligomer conjugates and other pro-polypeptide-oligomer conjugates are also provided.

WO-A-2007/007345 relates to a process for making an insulin-oligomer conjugate in a one-pot reaction by conjugation of insulin-ester with an activated oligomer wherein simultaneous deblocking and conjugation is carried out.

Ding Jin-Guo et al: "Expression of monomeric insulin precursor in Pichia pastoris and its conversion into monomeric B27 Lys destripeptide insulin by tryptic hydrolysis", Acta Biochimica et Biophysica Sinica, Blackwell Publishing, Inc., Malden, MA, US, Vol. 37, No. 4, 1 April 2005, pages 234-240, discuss the design and production of monomeric B27 Lys destripeptide insulin (B27 Lys DTrl) from its precursor expressed in *Pichia pastoris* through tryptic hydrolysis. The monomeric B27 Lys DTrl precursor (MIP) was purified from a cultured medium of *P*. *pastoris* by a combination of hydrophobic, size-exclusion, and ion-exchange chromatography and converted, by tryptic hydrolysis, to B27 Lys DTrl, which was then purified by ion-exchange chromatography to homogeneity as assessed by native gel electrophoresis, HPLC, amino acid composition, and electrospray mass-spectrometric analysis.

Kjeldsen T et al: "Secretory expression and characterization of insulin in Pichia pastoris", Biotechnology and Applied Biochemistry, Academic Press, US, Vol. 29, 1 January 1999, pages 79-86 report that the yeasts *Pichia pastoris* and *Saccharomycescerevisiae* have similar overall features regarding the secretory expression of insulin. The *S*. *cerevisiae* mating factor α (α-factor) prepro-leader facilitated the secretion of an insulin precursor, but not proinsulin expressed in *P*. *pastoris.* Synthetic prepro-leaders developed for the secretory expression of the insulin precursor in *S*. *cerevisiae* also facilitated the secretion of the insulin precursor expressed in *P. pastoris.* In contrast with *S*. *cerevisiae,* only insulin precursor and no unprocessed hyperglycosylated α-factor pro-leader/insulin precursor fusion protein was secreted from *P. pastoris.* A spacer peptide in the fusion protein increased the fermentation yield of the insulin precursor in *P. pastoris.* A synthetic prepro-leader, but not an α-factor prepro-leader lacking N-glycosylation sites, facilitated the secretion of the insulin precursor in *P. pastoris. P. pastoris* has a capacity for secretory expression of the insulin precursor that is equal to or better than that of *S*. *cerevisiae.*

Wang Yan et al: "Human insulin from a precursor overexpressed in the methylotrophic yeast Pichia pastoris and a simple procedure for purifying the expression product", Biotechnology and Bioengineering, Wiley & Sons, Hoboken, NJ, US, Vol. 73, No. 1, 5 April 2001, pages 74-79, teach the expression of an insulin precursor using *Pichia pastoris.* A transformant with a high copy number of the gene integrated into the chromosome was obtained by the dot-blotting method. In high-density fermentation using a simple culture medium composed mainly of salt and methanol, the expression level reached 1.5 g/L. A two-step method was established to purify the expression product from the culture medium with an overall recovery of about 80%. After tryptic transpeptidation, human insulin with full receptor binding capacity and biological activity was obtained.

The instant invention facilitates the making of insulin-oligomer conjugate from a specified precursor IP-X of formula Z-[B-Chain]-Q-[A-Chain], where Z is a leader where Z is a leader peptide sequence, B-Chain is B chain of human insulin or its analog, Q is a linker peptide sequence between the A and B chain, A-chain is the A chain of human insulin or its analog.

The precursor IP-X is conjugated with a specified oligomer at the LysB²⁹ position on the B-chain and the N-terminus amino acid of the leader peptide Z. The IP-X-oligomer conjugate is then subjected to protease treatment followed by purification to get an active insulin-oligomer conjugate.

The starting material is the fermented broth containing the precursor IP-X. The broth containing the IP-X is subjected to a combination of chromatography like ion-exchange, HPLC, RP-HPLC and crystallization to purify the IP-X.

The instant invention is a more simplified and economical in the making of an insulin-oligomer conjugate wherein several steps involved in obtaining pure insulin crystals in biologically active form are circumvented e.g, transpeptidation of the insulin precursor and cleaving the insulin precursor to get the active insulin and several chromatographic purification steps e.g., ion-exchange chromatography, HPLC and RP-HPLC to get the pure insulin crystals.

### SUMMARY OF THE INVENTION

The instant invention relates to a process of manufacturing insulin-oligomer conjugate IN-105 comprising, expressing the IN-105 precursor IP-F of formula G-A-V-R-[B-Chain]R-D-A-D-D-R-[A-Chain], treating IP-F with an activated oligomer which conjugates at the LysB²⁹ position of the IP-F and at the N-terminus amino acid Gly¹ of the leader peptide GAVR. The IP-F is subjected to conjugation with an oligomer having the general formula CO-(CH₂)₂-(OCH₂CH₂)₃-OCH₃. Specifically, the invention provides a process for the preparation of the insulin-oligomer conjugate IN-105 of formula insulin-CO-CH₂-CH₂-(OCH₂CH₂)₃-OCH₃, the process comprising:
i) cloning and expression of the synthetic polypeptide precursor IP-F having SEQ ID NO:2, in *Pichia pastoris;*
ii) fermentation of the *Pichia pastoris* clone expressing IPF;
iii) isolation and purification of the IP-F;
iv) conjugation of the IP-F with a succinimide derivative of C₁₄H₂₃NO₈ (CAS No. 622 405-78-1) at Lys-B²⁹ of the B chain in IP-F to obtain the IP-F oligomer conjugate IP-F-CO-CH₂-CH₂-(OCH₂CH₂)₃-OCH₃;
v) treatment of the IP-F-oligomer conjugate IP-F-CO-CH₂-CH₂-(OCH₂CH₂)₃-OCH₃ with a protease to get the active insulin-oligomer conjugate insulin-CO-CH₂-CH₂-(OCH₂CH₂)₃-OCH₃.

The instant invention involves the use of biosynthetic precursor sequence IP-F of formula G-A-V-R-[B-Chain]-R-D-A-D-D-R-[A-Chain]. The leader sequence G-A-V-R used in the present invention does not have any negatively charged amino acids as required for enhanced expression of insulin in yeast as disclosed in the literature. The process of the instant invention is a more simplified and economical in the making of an insulin conjugate wherein several steps of purification to obtain pure insulin in biologically active form are circumvented. The instant invention results in a product with no conjugation on the A chain or C-peptide (RDADDR) of IN-105 precursor IP-F and wherein the conjugation takes place on the B chain (LysB²⁹), and at Gly of the leader chain G-A-V-R which is then subjected to protease treatment to get insulin-oligomer conjugate IN-105. The overall cost of production of the conjugated insulin as a result of this process is minimized.

### DETAILED DESCRIPTION

IP-F represents a insulin-oligomer precursor of formula Z-[B-Chain]-Q-[A-Chain], where Z is a leader peptide sequence GAVR, B-chain is B(1-30) of human insulin, linker peptide Q is a linker peptide sequence RDADDR between the A and B chain, and A-chain is A(1-21) of human insulin. The IP-F of is cloned in-frame with the Mat-alpha signal peptide in the *Pichia pastoris* expression vector, pPIC9K. *Pichia pastoris* host strain, GS115 is transformed with the recombinant plasmid to obtain a *Pichia* clone expressing IPF. The secreted IP-F is treated with trypsin, carboxpeptidase B and N hydroxysuccinimide ester (activated oligomer) to yield IN105.

### SEQUENCE FOR THE IN-105 PRECURSOR IP-F.

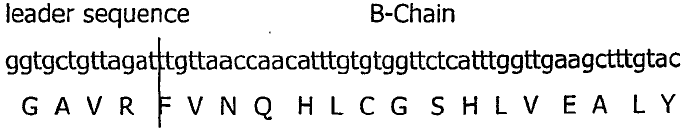

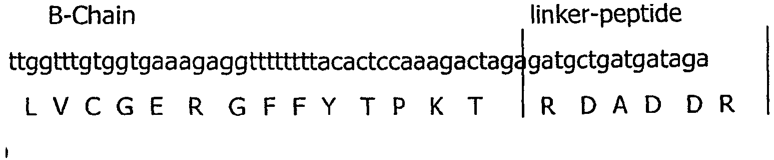

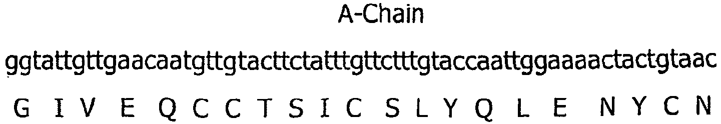

; Sequence : Codon optimized for expression in *Pichia pastoris*
Predicted Molecular weight: 6907.82 Da
Estimated pI value : 5.46
nucleotide (183 bp) and amino acid ( 61 aa )

The IN-105 precursor IP-F is secreted by *P. pastoris* into the culture medium. The broth is centrifuged and cells are separated from the supernatant. There are multiple options available for the capture of the precursor IP-F including Hydrophobic Interaction chromatography and Ion Exchange chromatography. In the instant invention, cation exchange chromatography and HIC is used to capture the IP-F.

Crystallization of the IN-105 precursor IP-F, removes any impurities carried through from the fermentation broth into the SP-Sepharose elution pool, this helps to reduce column fouling at the RP-HPLC-1 stage. Ammonium acetate salt, which is used to elute the product from the ion exchange column, is also removed by Crystallization. A pure crystalline precursor also helps in reducing cost in the subsequent conjugation step and increases the efficiency of the reaction. The crystalline form can be frozen and stored which would to be substantially stable for multiple days when stored at -20°C.

IN-105 is produced by a reaction wherein IP-F is first conjugated at B²⁹ Lysine using an activated oligomer to give IP-F-oligomer conjugate. The IP-F-oligomer conjugate is subjected to protease treatment, where the linker-peptide (RDADDR) and the leader sequence (GAVR) are cleaved to get the an active insulin-oligomer conjugate. The two proteases used are trypsin and carboxypeptidase B. As the LysB²⁹ is blocked by the oligomer, the probability of trypsin cleavage at LysB²⁹ is minimum. But the other probable sites for trypsin cleavage are c-terminus end of B-22 (Arg), C-1 (Arg) and C-6(Arg). A 2^{nd} protease (carboxypeptidase B) treatment is done to remove the free basic (Arg) amino acid from the B chain, where one extra Arg is attached with the B-30 (Thr) to get final product IN-105. The two protease treatment is carried out in a single operation at optimum reaction condition where yield was maximized with minimum product related impurities generated.

After the enzyme cleavage reaction is completed, the impurities may be separated from IN-105 e.g., the first RP-HPLC step. In the instant invention the impurities are removed when the first RP is run at low pH and final purification is done at high pH. A low loading and a gradient starting from 20 to 35 was carried out at 15 g/L of resin loading. The first RP-HPLC step gave a purity of .about.93-95%. There were a number of impurities which needed to be removed, where the impurities remaining after the first RP-HPLC step are purified by subjecting the elution pool of to another round of RP-HPLC. The final product of IN-105 had a purity to 97-98%.

### EXAMPLES

### Example 1

### EXPRESSION OF IN105 PRECURSOR IP-F IN PICHIA PASTORIS

pPIC9K/IP-F plasmid was digested with Bgl II and used to transform electrocompetent cells of *P*. *pastoris* GS115 (*his4*)*.* Regeneration mix was plated onto YNBD agar (1.34% Yeast Nitrogen Base without amino acids, 2% Dextrose, 2% agar) plates and incubated at 30°C for 48 hours. Colonies were grown in YPD (1% Yeast extract, 2% Peptone, 2% Dextrose) broth in micro titre plates along with appropriate controls. The plates were then stamped onto YPD agar (1% Yeast extract, 2% Peptone, 2% Dextrose, 2% agar) plates containing Geneticin (G418) and incubated at 30°C for 48 hours. Clones resistant to 1-3 mg/ml G418 were used for expression studies.
Genomic DNA was made from the selected recombinant Pichia clones using zymolyase enzyme for lysis. PCR was carried out using gene specific primers to confirm the integration of IP-F in the genome. GS115 host strain was used a negative control.

Expression studies in *P._pastoris* was carried out wherein the clones were grown at 30°C in BMGY (1% Peptone, 2% Yeast extract, 1.34% Yeast Nitrogen Base, 2% glycerol, 4 x 10⁻⁵% Biotin, 100mM phosphate buffer, pH 6.0). This was followed by induction with methanol in BMMY (1% Peptone, 2% Yeast extract, 1.34% Yeast Nitrogen Base, 0.5% Methanol, 4 x 10⁻⁵% Biotin, 100mM phosphate buffer, pH 6.0). Induction with methanol was carried out for a total of 3 days.
The crude supernatant from each of the clones was analysed on SDS-PAGE stained with Coomassie blue. IP-F was secreted as a ∼ 7 kDa protein.

The clones were analysed for secretion of IP-F for three days after methanol induction in shake flasks. An HPLC method was used for the analysis using Symmetry C18 column (4.6 x 250 mm, 300 A^{O}, 5 micron, Waters) and Buffers A (0.1 %TFA in water) and B (100 % Acetonitrile). The column is equilibrated with 25% Buffer A prior to sample injection. A programmed gradient is applied at a flow rate of 1 ml/min for the sample estimation : a linear gradient of 25% buffer A to 40 % buffer A is made in the first 15 minutes of the programmed gradient after the sample injection; 40 % Buffer A is maintained for the 15th and 16th minutes of the program; then the concentration of Buffer A is brought back to 25 % by using gradient between 16th to 18th minutes; the concentration of Buffer A is kept constant in the run for the last 5 minutes to equilibrate the column for the next run.

### Example 2

A loop full of culture from a single isolated colony grown on seed medium (1% Yeast extract, 0.5 peptone, 2% agar and 20 % dextrose monohydrate) is cultivated in 250 ml flask containing 50 ml BYYG medium (1% Peptone, 2% Yeast extract, 1.34% Yeast nitrogen base, 2% glycerol and 10% 1M phosphate buffer of pH 6.0) at 30+/-1 deg C and 230+/-10 rpm. After 48∼50 hrs of incubation the optical density measured at 600 nm reaches 10 +/- 2. The cells were resuspended in to 6 ml of production medium (1% Peptone, 2% Yeast extract, 1.34% Yeast nitrogen base, 0.5% methanol and 10% 1M phosphate buffer of pH 6.0) in 100 ml flask in order to make 50 % w/v cell suspension. Flasks were incubated at 30 deg C. 30 micro L of Methanol was added to all flasks every day from 2nd day. Assay on 4^{th} day was 0.069 g/L.

### Example 3

The seed flasks were prepared by cultivating frozen (-85 °C) cells of *Pichia* in 250 ml flask containing 50 ml growth medium (1% yeast extract, 2% peptone, 10% 1M phosphate buffer of pH 6.0, 0.67% Yeast nitrogen base and 0.1% glycerol) at 30 +/-1 deg C and 230 +/-10 rpm. After 20-28 hrs of incubation, OD (600nm) reaches 10-12. These cells were further cultivated in 2 L fermentor containing one liter fermentation medium consisting of 4 % glycerol, 0.0093% calcium sulfate, 1.82% potassium sulfate, 1.49% magnesium sulfate, 0.0413% potassium hydroxide. Fermentor was run at 30 deg C and pH of 5.0. The aeration rate was set to 0.1-1.0 wm. Agitation speed was adjusted to maintain the dissolved oxygen above 10%. Biomass was build up to 300-400 g/L by 50% glycerol feeding. Methanol was fed for induction. Assay on day 5 of methanol feeding is 0.76 g/L.

### EXAMPLE 4 (Selective conjugation)

IN-105 is prepared from the cell free fermentation broth containing the IN-105 precursor by steps comprising of,
**a)** SP-Sepharose column was equilibrated with buffer A(2C.V), pH of the cell free broth was adjusted to 4.0 with glacial acetic acid and the supernatant was loaded on to column at 45g/L of resin. Washing was done using Buffer A (1.5C.V) and product was eluted at 60% of Buffer B. The elution pool was concentrated 8 times and Yield was 95%. [Buffer A-10mM Ammonium acetate pH 4.0; Buffer B-1 M Ammonium acetate pH 4.0].
**b)** the elution pool containing the IN-105 precursor was taken in 1:1 dilution with water and the pH was adjusted to 5.0 with 10N NaOH. 0.4%(v/v) of phenol and 4%(v/v) of 0.3N zinc chloride was added. The mixture kept under cold condition for 6-8 hrs for crystallization. The recovery of IN-105 precursor was 95%.
**c)** 6g of the wet pellet containing the IN-105 precursor crystals was taken and 32ml of 500mM borate buffer of pH 8.1 was added to it. The pH was adjusted to 10.5 with 10 N NaOH. 370mg Oligomer dissolved in 11 ml of acetonitrile was added and the contents were stirred. Yield of total conjugated product was around 78%
**d)** Equal volume of the conjugated product from step(c) and water was taken. The pH of the solution was adjusted to 5.0 with glacial acetic acid then 0.4%(v/v) of phenol and 4%(v/v) of 0.3N zinc chloride was added was added to the mixture and the pH was adjusted to 5.1 with 1N NaOH. The mixture was kept over night at 4°C for crystallization. Yield in this step was 90%,
**e)** the wet crystal pellet from step (d) was solubilized in 0.5M tris base solution to make the a product concentration of 16g/l. The pH of the solution was made 7.4 using 1N NaOH. Then 0.027mM of trypsin and 0.3x10⁻³mM of carboxypeptidase B was added to the reaction mixture and kept at 24°C for 12 hrs. The product formed was IN-105 with a step yield of 80%,
**f)** A column packed with resin of particle size: 10-15µ, pore size: 120 Å was equilibrated using 15%B, the reaction mixture from step(e) was diluted 1:10 and pH was adjusted to 7.0 before loading, and washing was done with 15%B. Elution was done using 15 to 25%B over 20Cvs. Fractions of 95% purity were got [Buffer A: 10mM Sodium acetate pH 7.0; Buffer B:100% Acetonitrile].
**g)** The eluent from step(f) is further diluted and pH is adjusted to 8.5 before loading, and washing was done with 20%B. Elution was done using 20 to 35%B over 15CVs. Fractions of 97% purity were got [Buffer: 100mM Tris pH 8.5; 20mM Magnesium Chloride; Buffer B:Acetonitrile]
**h)** The elution pool from step (g) was taken and the concentration of IN-105 was brought down to 6mg/ml by dilution with water. The pH of the sample was adjusted to 4.5 with glacial acetic acid. 0.6%(v/v)phenol and 4%(v/v) of 0.3N zinc chloride were added and pH was finally made to 5 and the mixture was kept at 4°C overnight to form crystals. The crystals were collected after centrifugation with a step yield of 90%.

### EXAMPLE 5 (Selective conjugation)

IN-105 is prepared from the cell free fermentation broth containing the IN-105 precursor by steps comprising of,
**a)** Taking the Cell free broth at pH 7 was loaded onto a column packed with PLRP S 50 -70 MICRON. The broth was made to 5% MeCN and loaded onto the column, which was equilibrated with 10mM Sodium acetate pH 7.0 and 5%MeCN. A recovery of 90% from 4 g/L loading was got,
**b)** the elution pool containing the IN-105 precursor was taken in 1:1 dilution with water and the pH was adjusted to 5.0 with 10N NaOH. 0.4%(v/v) of phenol and 4%(v/v) of 0.3N zinc chloride was added. The mixture kept under cold condition for 6-8 hrs for crystallization. The recovery of IN-105 precursor was 95%.
**c)** 20g of the wet pellet containing IN-105 precursor crystals was taken and 92ml of 500mM borate buffer of pH 8.1 was added. The pH was adjusted to 10.5 using 10 N NaOH; 1.13g of Oligomer dissolved in 40ml acetonitrile was added while stirring the reaction mixture. Yield of total conjugated product was 77%.
**d)** Equal volume of the conjugated product from step(c) and water was taken. The pH of the solution was adjusted to 5.0 with glacial acetic acid then 0.4%(v/v) of phenol and 4%(v/v) of 0.3N zinc chloride was added was added to the mixture and the pH was adjusted to 5.1 with 1N NaOH. The mixture was kept over night at 4°C for crystallization. Yield in this step was 90%,
**e)** the wet crystal pellet from step (d) was solubilized in 0.5M tris base solution to make a product concentration of 20g/l. The pH of the solution was adjusted to 7.4 with 1N NaOH. 0.02mM trypsin and 13x10⁻³mM of carboxypeptidase B was added to the reaction mixture. At the end of 12 hrs product formed was IN-105 with a step yield of 85%,
**f)** A column packed with resin of particle size: 10-15g, pore size: 120 Å was equilibrated using, 10% of buffer B, the reaction mixture from step(e) was diluted 1:10 with concentration of MeCN at 33% and pH adjusted to 3.5, washing was done with 15%B. Elution was done using step gradient. Yield of 58.6% were got, Buffer A:10 mM Sodium Acetate pH 7.0, Buffer B: 100 % Acetonitrile.
**g)** the eluent from step(f) is further diluted and pH is adjusted to 7.5 before loading, and washing was done with 20% of buffer B. Elution was done using 22 to 32% of buffer B over 20CVs. Fractions of 97% purity were got [BufferA:100mM Tris pH 8.5;20mM Magnesium Chloride; Buffer B: Acetonitrile].
**h)** The elution pool from step (g) was taken and the concentration of IN-105 was brought down to 6mg/ml by dilution with water. The pH of the sample is adjusted to 4.5 with glacial acetic acid. 0.6%(v/v) of phenol and 4%(v/v) of 0.3N zinc chloride were added and pH was finally made to 5 and the mixture was kept at 4°C overnight to form IN-105 crystals. The crystals were collected after centrifugation with a step yield of 90%.

### EXAMPLE 6 (Exhaustive conjugation)

**a)** SP-Sepharose column was equilibrated with bufferA(2C.V), pH of the cell free broth was adjusted to 4.0 with glacial acetic acid and the supernatant was loaded on to column at 20g/l load. Washing was done using Buffer A (1.5C.V) and product was eluted at 0 to 100%B in 10C.V. The elution pool was concentrated 8 times and Yield was 98%. [Buffer A-10mM Ammonium acetate pH 4.0; Buffer B-1 M Ammonium acetate pH 4.0].
**b)** the elution pool containing the IN-105 precursor was taken in 1:1 dilution with water and the pH was adjusted to 5.0 with 10N NaOH. 0.4%(v/v) of phenol and 4%(v/v) of 0.3N zinc chloride was added. The mixture kept under cold condition for 6-8 hrs for crystallization. The recovery of IN-105 precursor was 95%.
**c)** the wet crystal pellet was solubilized in DMSO to make the concentration of the product 60g/ml. To the reaction mixture 0.001 % (v/v) of triethyl amine was added. Oligomer was solubilized in tetrahydrofuran(THF) at a concentration of 0.4 M and added to the reaction mixture. At the end of 15 hrs exhaustively conjugated product with a yield of 80% was recovered.
**d)** equal volume of the conjugated product from step(c) and water was taken. The pH of the solution was adjusted to 5.0 with glacial acetic acid then 0.4%(v/v) of phenol and 4%(v/v) of 0.3N zinc chloride was added was added to the mixture and the pH was adjusted to 5.1 with 1N NaOH. The mixture was kept over night at 4°C for crystallization. Yield in this step was 90%
**e)** the wet crystal pellet from step (d) was solubilized in 0.5M tris base solution to make a product concentration of 25g/l. The pH of the solution was raised to 7.4 with 1N NaOH. 0.02mM trypsin and .3x10⁻³ carboxypeptidase was added to the reaction mixture. At the end of 14 hrs the IN-105 formed had a step yield of 80%
**f)** A column packed with resin of particle size:10-15µ, pore size: 120 Å was equilibrated using 15% of buffer B; the reaction mixture from step(e) was diluted 1:10 and pH was adjusted to 7.0 before loading. Washing was done with 15% of buffer B and elution was done using 27 to 37% of buffer B over 20Cvs. Fractions of 90% purity were got BufferA: 100mM Tris pH 8.0; 20mMMgCl₂, pH 8.5. Buffer B:100% Acetonitrile.
**g)** The eluent from step(f) is further diluted and pH is adjusted to 9.0 before loading, and washing was done with 20% of buffer B. Elution was done using 20 to 35% of buffer B over 15CVs. Fractions of 97% purity were got [BufferA:100mM Tris pH 9.0; 20mM Magnesium Chloride; Buffer B: Acetonitrile].
**h)** The elution pool from step (g) was taken and the concentration of IN-105 was brought down to 6mg/ml by dilution with water. The pH of the sample is adjusted from 8.3 to 4.5 with glacial acetic acid. 0.6%(v/v) of phenol and 4%(v/v) of 0.3N zinc chloride were added and pH was finally made to 5 and the mixture was kept at 4°C overnight to form crystals. The crystals were collected after centrifugation with a step yield of 90%.

### EXAMPLE 7 (Exhaustive conjugation)

**a)** SP-Sepharose column was equilibrated with bufferA(2C.V), pH of the cell free broth was adjusted to 4.0 with glacial acetic acid and the supernatant was loaded on to column at 45g/l load. Washing was done using Buffer A (1.5C.V) and product was eluted at 60% B. The elution pool was concentrated 8 times and Yield was 95%, [Buffer A-10mM Ammonium acetate pH 4.0; Buffer B-1 M Ammonium acetate pH 4.0]
**b)** the of elution pool containing the IN-105 precursor was taken in 1:1 dilution with water and the pH was adjusted to 5.0 with 10N NaOH. 0.4%(v/v) of phenol and 4%(v/v) of 0.3N zinc chloride was added. The mixture kept under cold condition for 6-8 hrs for crystallization. The recovery of IN-105 precursor was 95%,
**c)** the wet crystal pellet was solubilized in DMSO to make the concentration of the product 60g/ml. To the reaction mixture 0.001 % (v/v) of triethyl amine was added. Oligomer was solubilized in tetrahydrofuran(THF) at a concentration of 0.4 M and added to the reaction mixture. At the end of 15 hrs exhaustively conjugated product with a yield of 80% was recovered.
**d)** Equal volume of the conjugated product from step(c) and water was taken. The pH of the solution was adjusted to 5.0 with glacial acetic acid then 0.4%(v/v) of phenol and 4%(v/v) of 0.3N zinc chloride was added was added to the mixture and the pH was adjusted to 5.1 with 1N NaOH. The mixture was kept over night at 4°C for crystallization. Yield in this step was 90%,
**e)** the wet crystal pellet from step (d) was solubilized in 0.5M Tris base solution to make a product concentration of 25g/l. The pH of the solution was raised to 7.4 with 1N NaOH, to the reaction mixture 0.02mM trypsin and .3x10⁻³ carboxypeptidase B were added. At the end of 14 hrs the IN-105 formed had a step yield 80%
**f)** A column packed with resin of particle size:10-15µ, pore size: 120 Å was equilibrated using 10% of buffer B, the reaction mixture from step(e) was diluted 1:10 with concentration of MeCN at 15% and pH adjusted to 3.5 and load filtered through 5 micron filter ; washing was done with 15%B. Elution was done using 17 to 23% of buffer B over 20Cvs. 94.2% pure protein with a yield of 77% were got. [Buffer A: 250 mM Acetic Acid, Buffer B: Acetonitrile]
**g)** the eluent from step(f) is further diluted and pH is adjusted to 9.0 before loading, and washing was done with 20% of buffer B. Elution was done using 22 to 32%B over 15CVs. Fractions of 97% purity were got [BufferA:100mM Tris pH 9.0; 20mM Magnesium Chloride; Buffer B: Acetonitrile]
**h)** The elution pool from step (g) was taken and the concentration of IN-105 was brought down to 6mg/ml by dilution with water. The pH of the sample is adjusted from 8.3 to 4.5 with glacial acetic acid. 0.6%(v/v) of phenol and 4%(v/v) of 0.3N zinc chloride were added and pH was finally made to 5 and the mixture was kept at 4°C overnight to form IN-105 crystals. The crystals were collected after centrifugation with a step yield of 90%.

## Claims

1. A process for the preparation of the insulin-oligomer conjugate IN-105 of formula insulin-CO-CH₂-CH₂-(OCH₂CH₂)₃-OCH₃, the process comprising:
i) cloning and expression of the synthetic polypeptide precursor IP-F having SEQ ID NO:2, in *Pichia pastoris;*
ii) fermentation of the *Pichia pastoris* clone expressing IPF;
iii) isolation and purification of the IP-F;
iv) conjugation of the IP-F with a succinimide derivative of C₁₄H₂₃NO₈ (CAS No. 622 405-78-1) at Lys-B²⁹ of the B chain in IP-F to obtain the IP-F oligomer conjugate IP-F-CO-CH₂-CH₂-(OCH₂CH₂)₃-OCH₃;
v) treatment of the IP-F-oligomer conjugate IP-F-CO-CH₂-CH₂-(OCH₂CH₂)₃-OCH₃ with a protease to get the active insulin-oligomer conjugate insulin-CO-CH₂-CH₂-(OCH₂CH₂)₃-OCH₃.

2. A process of claim 1, wherein the precursor IP-F is isolated from the broth by ion exchange chromatography followed by crystallization.

3. A process of claim 2, wherein the crystallization is carried out in phenol and ZnCl₂.

4. A process of claim 1, 2 or 3, wherein the conjugation of IP-F with the succinimide derivative of C₁₄H₂₃NO₈ (CAS No. 622 405-78-1) is carried out in one or more solvents selected from borate buffer, acetonitrile, DMSO.

5. A process of claim 1 to 4, wherein the IP-F oligomer conjugate IP-F-CO-CH₂-CH₂-(OCH₂CH₂)₃-OCH₃ is further subjected to crystallization.

6. A process of any of the preceding claims, wherein the protease is selected from trypsin or carboxypeptidase B or mixture thereof.

7. A process of any of the preceding claims, wherein the active insulin-oligomer conjugate insulin-CO-CH₂-CH₂-(OCH₂CH₂)₃-OCH₃ is purified by RP-HPLC and crystallization.

## Patentansprüche

1. Verfahren zur Herstellung des Insulin-Oligomer-Konjugats IN-105 der Formel Insulin-CO-CH₂-CH₂-(OCH₂CH₂)₃-OCH₃, wobei das Verfahren folgendes umfasst:
i) Klonen und Exprimieren des synthetischen Polypeptid-Präkursors IP-F mit der SEQ ID Nr:2 in *Pichia pastoris;*
ii) Fermentation des *Pichia pastoris-Klons,* der IPF exprimiert;
iii) Isolieren und Aufreinigen des IP-F;
iv) Konjugieren des IP-F mit einem Succinimid-Derivat von C₁₄H₂₃NO₈ (CAS Nr. 622 405-78-1) an Lys-B²⁹ der B-Kette in IP-F, um das IP-F-Oligomer-Konjugat IP-F-CO-CH₂-CH₂-(OCH₂CH₂)₃-OCH₃ zu erhalten;
v) Behandeln des IP-F-Oligomer-Konjugats IP-F- CO-CH₂-CH₂-(OCH₂CH₂)₃-OCH₃ mit einer Protease, um das aktive Insulin-Oligomer-Konjugat Insulin-CO-CH₂-CH₂-(OCH₂CH₂)₃-OCH₃ zu erhalten.

2. Verfahren nach Anspruch 1, wobei der Präkursor IP-F isoliert ist von der Nährlösung durch lonenaustauschchromatographie gefolgt von Kristallisation.

3. Verfahren nach Anspruch 2, wobei die Kristallisation in Phenol und ZnCl₂ ausgeführt wird.

4. Verfahren nach Anspruch 1, 2 oder 3, wobei die Konjugation von IP-F mit dem Succinimid-Derivat von C₁₄H₂₃NO₈ (CAS Nr. 622 405-78-1) ausgeführt wird in einem oder mehreren Lösungsmitteln, die ausgewählt sind aus Boratpuffer, Acetonitril, DMSO.

5. Verfahren nach einem der Ansprüche 1 bis 4, wobei das IP-F-Oligomer-Konjugat IP-F- CO-CH₂-CH₂-(OCH₂CH₂)₃-OCH₃ ferner Kristallisation ausgesetzt wird.

6. Verfahren nach einem der vorstehenden Ansprüche, wobei die Protease ausgewählt ist aus Trypsin oder Carboxypeptidase B oder einem Gemisch davon.

7. Verfahren nach einem der vorstehenden Ansprüche, wobei das aktive Insulin-Oligomer-Konjugat Insulin-CO-CH₂-CH₂-(OCH₂CH₂)₃-OCH₃ aufgereinigt wird durch RP-HPLC und Kristallisation.

## Revendications

1. Procédé de préparation d'un conjugué insuline-oligomère IN-105 de formule insuline-CO-CH₂-CH₂-(OCH₂CH₂)₃-OCH₃, le procédé comprenant :
i) le clonage et l'expression du précurseur polypeptidique synthétique IP-F possédant SEQ ID NO:2, chez *Pichia pastoris ;*
ii) la fermentation du clone *Pichia pastoris* exprimant l'IPF ;
iii) l'isolation et la purification de l'IP-F ;
iv) la conjugaison de l'IP-F avec un dérivé de succinimide C₁₄H₂₃NO₈ (Numéro CAS 622 405-78-1) au Lys-B²⁹ de la chaine B dans IP-F pour obtenir le conjugué IP-F-oligomère IP-F-CO-CH₂-CH₂-(OCH₂CH₂)₃-OCH₃ ;
v) le traitement du conjugué IP-F-oligomère IP-F-CO-CH₂-CH₂-(OCH₂CH₂)₃-OCH₃ avec une protéase pour obtenir le conjugué actif insuline-oligomère insuline-CO-CH₂-CH₂-(OCH₂CH₂)₃-OCH₃.

2. Procédé selon la revendication 1, dans lequel le précurseur IP-F est isolé à partir du bouillon à l'aide de la chromatographie échangeuse d'ions suivie de la cristallisation.

3. Procédé selon la revendication 2, dans lequel la cristallisation est effectuée dans du phénol et du ZnCl₂.

4. Procédé selon la revendication 1, 2 ou 3, dans lequel la conjugaison de l'IP-F avec le dérivé de succinimide C₁₄H₂₃NO₈ (Numéro CAS 622 405-78-1) est effectuée dans un ou plusieurs solvants choisis parmi le tampon de borate, l'acétonitrile, le DMSO.

5. Procédé selon la revendication 1 à 4, dans lequel le conjugué IP-F-oligomère IP-F-CO-CH₂-CH₂-(OCH₂CH₂)₃-OCH₃ est en outre sujet à la cristallisation.

6. Procédé selon l'une quelconques des revendications précédentes, dans lequel la protéase est choisie parmi la trypsine ou la carboxypeptidase B ou un mélange de celles-ci.

7. Procédé selon l'une quelconques des revendications précédentes, dans lequel le conjugué actif insuline-oligomère insuline-CO-CH₂-CH₂-(OCH₂CH₂)₃-OCH₃ est purifié par CLHP-PI et par cristallisation.
